# EUROPEAN PATENT APPLICATION

(11) **EP 1 932 826 A1**
(43) Date of publication of application: **18.06.2008**
(21) Application number: 06746495.8
(22) Date of filing: 17.05.2006
(51) Int. Cl.: C07C 67/313, C07C 65/24, C07C 69/94, C07C 213/02, C07C 217/22, C07C 255/54, C07D 303/22, C07B 53/00

(54) **METHOD FOR PRODUCING AMINOALCOHOL DERIVATIVE HAVING BIPHENYL GROUP**

(30) Priority: 19.05.2005 JP 2005147178
(71) Applicant: Kissei Pharmaceutical Co., Ltd., Matsumoto-shi Nagano 399-8710 (JP)
(72) Inventor: KASAI, Kiyoshi c/o Central Research Laboratories,, Kashiwabara, Nagano, 399- 8304 (JP); KUBOTA, Minoru c/o Process Chemistry Research,, Technoport, Mikuni-ho Fukui, 913-0038 (JP); SUZUKI, Ritsu c/o Central Research Laborato, Hotaka-Kashiwabara Nagano;3998304 (JP); OZAWA, Tetsuji c/o Central Research Laboratories,, Kashiwabara, Nagano, 399-8304 (JP); ISHIKAWA, Takehiro c/o Central Research Laboratorie, Hotaka-Kashiwabara,Nagano, 399-8304 (JP); KOBAYASHI, MakotoI c/o Central Research Laboratorie, Hotaka-Kashiwabara,Azumino-shi, Nagano 399-8304 (JP); SONEHARA, Junichi c/o Process Chemistry Research,, Technoport, Mikuni-cho Sakai-shi, Fukui, 913-0038 (JP); KOBAYASHI, Masahiro c/o Process Chemistry Research,, Technoport, Mikuni-cho Sakai-shi, Fukui, 913-0038 (JP)
(74) Representative: Hayes, Adrian Chetwynd
(86) International application number: PCT/JP2006/309792
(87) International publication number: WO 2006/123672

(57) **Abstract**

The present invention provides an industrial method for preparing aminoalcohol derivatives (I) having a biphenyl group, biphenyloxyacetaldehyde derivatives which are used therefore, preparing methods thereof, and intermediates used for the preparing method.

The present invention also provides a preparing method including processes for preparing a 3-substituted biphenylcarboxylic acid derivative represented by the following general formula: 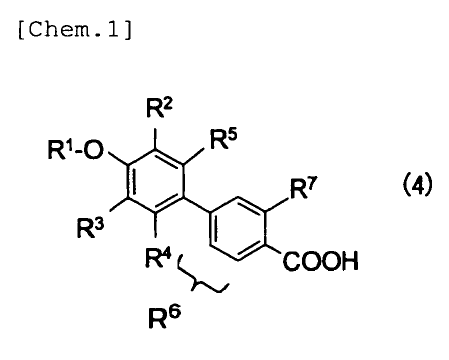 by allowing a 3-fluorobiphenylcarboxylic acid derivative prepared from a 3-fluorobiphenylcarbonitrile derivative which can be prepared by allowing a 4-halophenol derivative to react with a 2-fluorobenzonitrile derivative which is substituted by a leaving group at 4-position to react with an organometallic reagent.

## Description

### Technical Field

The present invention relates to a biphenyl derivative which is useful as a preparing intermediate for a medicament and a method for preparing the same, and a method for preparing an aminoalcohol derivative having a biphenyl group using the same. More particularly, the present invention relates to a biphenyl derivative which can be used for preparing aminoalcohol derivative (I) having a biphenyl group represented by the following general formula: 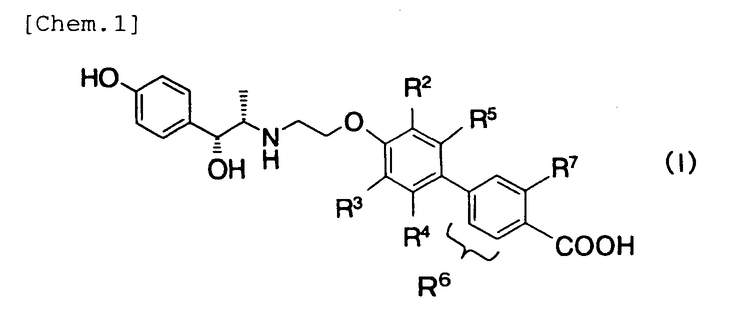 wherein R² to R⁵ represent independently a hydrogen atom, a fluorine atom, a chlorine atom, a lower alkyl group, a fluoro-lower alkyl group or a lower alkoxy group, R⁶ represents a fluorine atom, a chlorine atom, a lower alkyl group, a fluoro-lower alkyl group, a cycloalkyl group, a lower alkoxy group or an aryloxy group which may have a substituent, R⁷ represents a lower alkyl group, a fluoro-lower alkyl group, a cycloalkyl group or an aryl group which may have a substituent, and "}R⁶" indicates that R6 exists at m-position and/or p-position of R⁷ (when two R⁶ exist, these may be different, the same hereinafter.); and a method for preparing the same, and a method for preparing aminoalcohol derivative (I) using the biphenyl derivative.

### Background Art

Aminoalcohol derivative (I) having a biphenyl group shows a stimulating activity on β₃-adrenoceptor and can be used for treating or preventing obesity, diabetes mellitus, hyperlipidemia, depression, urinary dysfunctions, diseases caused by gallstones or biliary tract hypermotility, diseases caused by gastrointestinal hypermotility or the like (see Patent Reference 1).
As one of methods for preparing aminoalcohol derivative (I) having a biphenyl group, Patent Reference 1 describes a method wherein after reductive amination of a biphenyloxyacetoaldehyde derivative and an aminoalcohol derivative, the obtained product is hydrolyzed. However, anymethod for preparing a biphenyloxyacetaldehyde derivative substituted by an alkyl group, a carboxy group or the like at a specific position is not described in Patent Reference. In addition, as a preparing intermediate of a biphenyloxyacetaldehyde derivative, the corresponding alcohol compound or acetal compound is described, but because neither of these is easily crystallized, it is not easy to handle, and it is not preferable as an industrial preparing intermediate.
In the same reference, though a method for preparing aminoalcohol derivative (I) including a process of conducting alkylation of the amine after converting an alcohol part of biphenyloxyethanol into a suitable leaving group has been described, there is a problem with a low yield.

Furthermore, in the same reference, as another method for preparing aminoalcohol derivative (I), a method including a process wherein a phenylboronic acid derivative is subjected to cross-coupling reaction with an aminoethoxyhalobenzene derivative in the presence of a heavy metal catalyst such as palladium or the like is described. However, since a complicated product is obtained by this cross-coupling reaction, multiple times chromatographies are needed to isolate a target substance. Patent Reference 1: International Publication No. WO2004/072016 pamphlet

### Disclosure of the Invention

### Problem to be solved by the Invention

The object of the present invention is to provide an industrial method for preparing the above aminoalcohol derivatives (I) having a biphenyl group, biphenyloxyacetaldehyde derivatives used for the method and a method for preparing the same, and intermediates used for the preparing method.

### Means of solving the Problems

As a result that the present inventors have studied earnestly to solve the above problem, it was found that a biphenyloxyacetoaldehyde derivative can be prepared in good yield by employing a preparing method including a process for preparing a biphenylcarbonitrile derivative by subjecting a 4-halophenol derivative to cross-coupling reaction with a 2-fluorobenzonitrile derivative which is substituted by a leaving group at 4-position and a process for preparing a 3-substituted biphenylcarboxylic acid derivative by subjecting a biphenylcarboxylic acid derivative which is prepared from a biphenylcarbonitrile derivative to aromatic nucleophilic substitution reaction against the fluorine atom at 3-position, a handling becomes easy by using an epoxide which is easily crystallized as a preparing intermediate of a biphenyloxyacetoaldehyde derivative, and a high quality biphenyloxyacetoaldehyde can be prepared without purification such as recrystallization, column chromatography or the like, thereby forming the bases of the present invention.
That is, a subject matter of the present invention relates to;
a method for preparing biphenyloxyacetoaldehyde derivative (9) represented by the general formula: 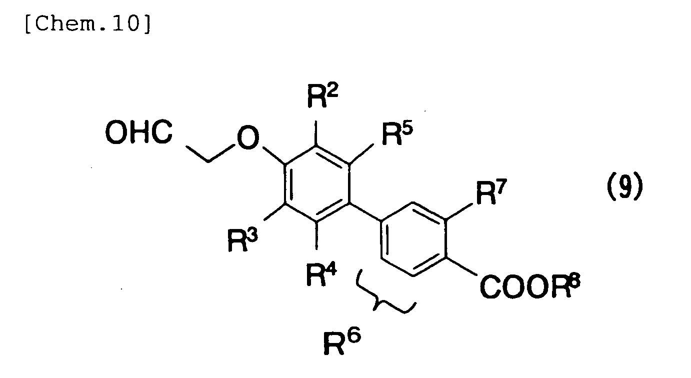 wherein R² to R⁵ represent independently a hydrogen atom, a fluorine atom, a chlorine atom, a lower alkyl group, a fluoro-lower alkyl group or a lower alkoxy group, R⁶ represents a fluorine atom, a chlorine atom, a lower alkyl group, a fluoro-lower alkyl group, a cycloalkyl group, a lower alkoxy group or an aryloxy group which may have a substituent, R⁷ represents a lower alkyl group, a fluoro-lower alkyl group, a cycloalkyl group or an aryl group which may have a substituent, R⁸ represents a lower alkyl group or an arylmethy group which may have a substituent, and "}R⁶" indicates that R⁶ exists at m-position and/or p-position of R⁷ (when two R⁶ exist, these may be different), which comprises:
Process 1 for preparing 3-fluorobiphenylcarbonitrile derivative (2) represented by the general formula: 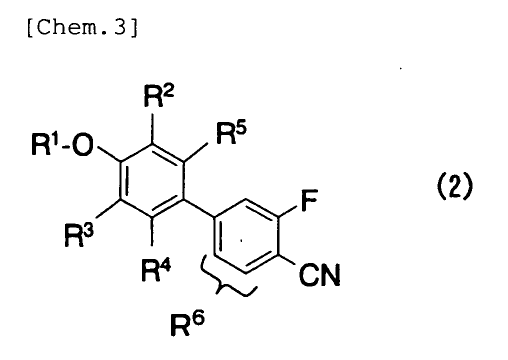 wherein R¹ represents a hydroxy-protective group, R² to R⁶ have the same meanings as defined above, and "}R⁶" indicates that R⁶ exists at m-position and/or p-position of the fluorine atom (when two R⁶ exist, these may be different), by allowing halophenol derivative (1) represented by the following general formula: 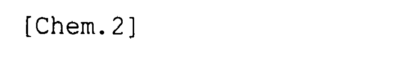 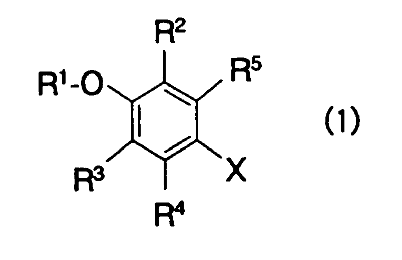 wherein R¹ to R⁵ have the same meanings as defined above, and X represents a chlorine atom, a bromine atom or an iodine atom, to react with a 2-fluorobenzonitrile derivative which is substituted by a leaving group at 4-position;
Process 2 for preparing 3-fluorobiphenylcarboxylic acid derivative (3) represented by the general formula: 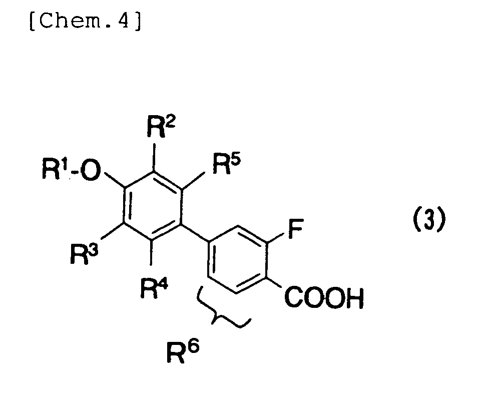 wherein R¹ to R⁶ have the same meanings as defined above, by hydrolysis of 3-fluorobiphenylcarbonitrile derivative (2);
Process 3 for preparing 3-substituted biphenylcarboxylic acid derivative (4) represented by the general formula: 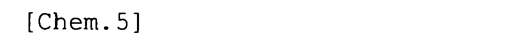 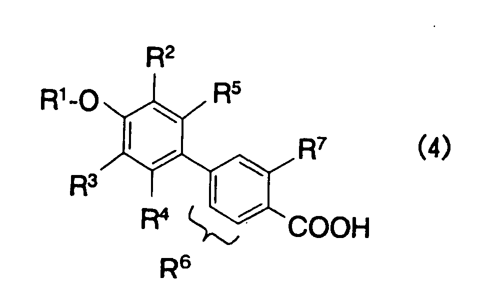 wherein R¹ to R⁷ have the same meanings as defined above, by allowing 3-fluorobiphenylcarboxylic acid derivative (3) to react with a lower alkyl metal reagent, a fluoro-lower alkyl metal reagent, a cycloalkyl metal reagent or an aryl metal reagent which may have a substituent;
Process 4 for preparing 3-substituted biphenylcarboxylate (5) represented by the general formula: 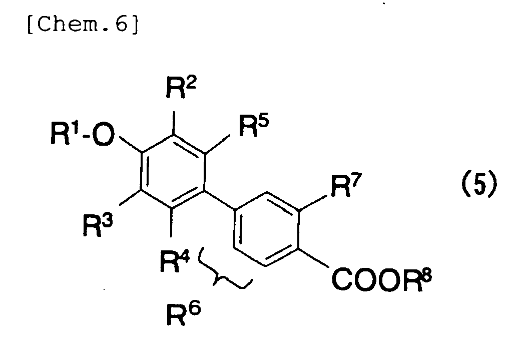 wherein R¹ to R⁸ have the same meanings as defined above, by esterification of 3-substituted biphenylcarboxylic acid derivative (4);
Process 5 for preparing 4'-hydroxybiphenylcarboxylate (6) represented by the general formula: 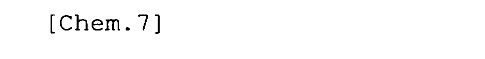 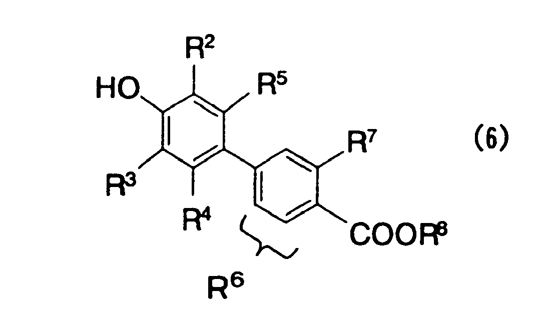 wherein R² to R⁸ have the same meanings as defined above, by removal of a hydroxy-protective group of 3-substituted biphenylcarboxylate (5);
Process 6 for preparing 4'-epoxypropoxybiphenylcarboxylate (7) represented by the general formula: 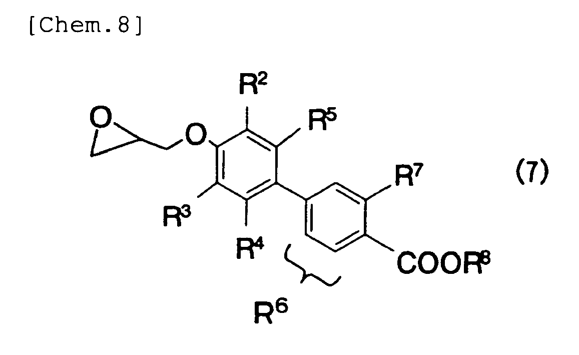 wherein R² to R⁸ have the same meanings as defined above, by allowing 4'-hydroxybiphenylcarboxylate (6) to react with an epihalohydrin;
Process 7 for preparing 4'-dihydroxypropoxybiphenylcarboxylate (8) represented by the general formula: 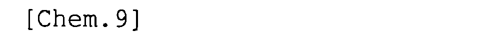 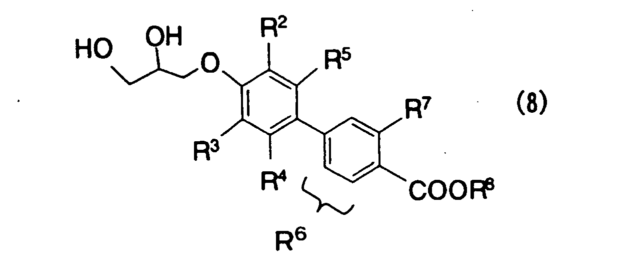 wherein R² to R⁸ have the same meanings as defined above, by cleavage reaction of 4'-epoxypropoxybiphenylcarboxylate (7) to form a diol; and
Process 8 for preparing biphenyloxyacetoaldehyde derivative (9) by oxidation of 4'-dihydroxypropoxybiphenylcarboxylate (8); a method for preparing aminoalcohol derivative (I): 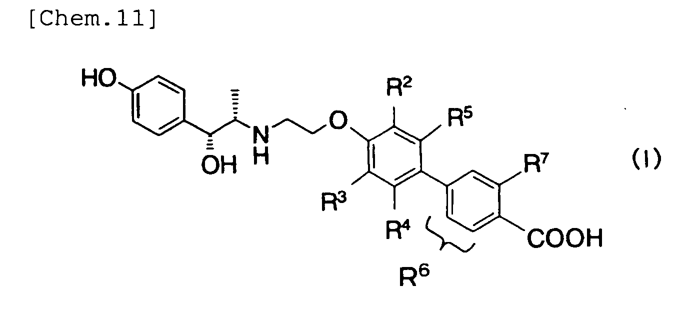 wherein R² to R⁷ have the same meanings as defined above, which comprises subjecting biphenyloxyaldehyde derivative (9) represented by the following general formula: 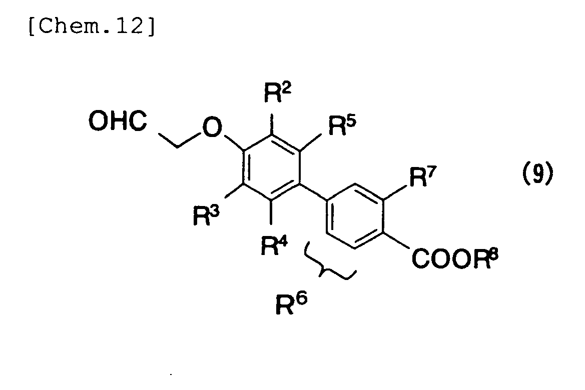 wherein R² to R⁷ have the same meanings as defined above, and R⁸ represents a lower alkyl group or an arylmethyl group which may have a substituent, to reductive amination with aminoalcohol derivative (II) represented by the general formula: 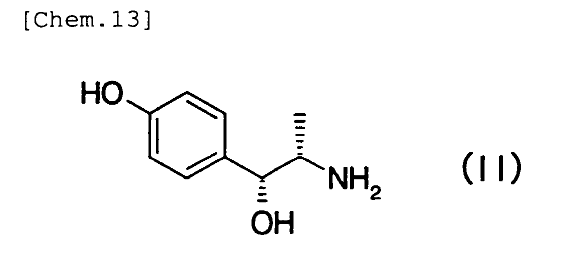 , and subsequently by hydrolysis; and
   compounds (2) to (9) used for the above preparing method.

### Effect of the Invention

According to a method for preparing of the present invention, aminoalcohol derivatives (I) having a biphenyl group can be prepared in good yield, in addition, a epoxypropoxy-biphenylcarboxylic acid derivative which is the intermediate used for the method for preparing is easy to treat because of being a crystalline compound.

### Best Mode to practice the Invention

In the present invention, the term "halo" or "halogen" means fluorine, chlorine, bromine or iodine.
The term "lower" means a straight-chained or branched hydrocarbon group having 1 to 6 carbon atoms. As lower alkyl, for example, methyl, ethyl, *n-*propyl, isopropyl, *n-*butyl, isobutyl, *sec-*butyl, *tert-*butyl, *n-*pentyl, isopentyl, neopentyl, *sec-*pentyl, *tert-*pentyl, *n-*hexyl and the like can be illustrated. The term "fluoro-lower alkyl" means a group wherein one and more hydrogen atoms of lower alkyl are substituted by fluorine atoms. For example, fluoromethyl, difluoromethyl, trifluoromethyl, 2,2,2-trifluoroethyl and the like can be illustrated.
The term "cycloalkyl" means a 3 to 7-membered cyclic hydrocarbon group which may have lower alkyl. For example, cyclopropyl, methylcyclopropyl, cyclobutyl, cyclopentyl, cyclohexyl, dimethylcyclohexyl, cycloheptyl and the like can be illustrated.
As lower alkoxy, for example, methoxy, ethoxy, *n*-propoxy, isopropyloxy, *n*-butoxy, isobutyloxy, *sec*-butoxy, *tert*-butoxy, *n-*pentyloxy, isopentyloxy, neopentyloxy, *sec*-pentyloxy, *tert*-pentyloxy, *n*-hexyloxy group and the like can be illustrated.
As aryl, phenyl and naphthyl can be illustrated.
As a substituent which an aryl may have, for example, a group selected from a group consisting of halogen, lower alkyl, lower alkoxy or the like can be illustrated.
As a metal of a lower alkyl metal reagent, a fluoro-lower alkyl metal reagent, a cycloalkyl metal reagent or an aryl metal reagent which may have a substituent, for example, a magnesium halide, lithium, a zinc halide, copper-lithium complex and the like can be illustrated.
As an alkali metal, lithium, sodium and potassium can be illustrated.
The method for preparing of the present invention is described in each process as follows.

### (Process 1)

This is a process for preparing 3-fluorobiphenylcarbonitrile derivative (2) by allowing halophenol derivative (1) to react with a 2-fluorobenzonitrile which is substituted by a leaving group at 4-position.

As a hydroxy-protective group R¹ of halophenol derivative (1), for example, a benzyl group which may have a substituent such as a lower alkyl group, a lower alkoxy group or the like; a silyl group such as a triethylsilyl group or the like; a group forming a straight-chained or cyclic acetal such as a methoxyethoxymethyl group, a tetrahydropranyl group or the like; and the like can be illustrated.

As R², a straight-chained alkyl group, especially, a methyl group, an ethyl group or a *n-*propyl group is preferable.

As R³, a straight-chained alkyl group, especially, a methyl group, an ethyl group or a *n-*propyl group is preferable.

As R⁴, a hydrogen atom, a methyl group, an ethyl group, a *n-*propyl group or an isopropyl group, especially, a hydrogen atom is preferable.

As R⁵, a hydrogen atom, a methyl group, an ethyl group, a *n*-propyl group or an isopropyl group, especially, a hydrogen atom is preferable.

As X, a bromine atom or an iodine atom is preferable.

As halophenol derivative (1), a commercially available one or one prepared by a well-known method or optionally modified the same can be used.

As a leaving group of a 2-fluorobenzonitrile derivative having the leaving group at 4-position, for example, a halogen atom, a lower alkylsulfonyloxy group, a halo-lower alkylsulfonyloxy group or an arylsulfonyloxy group which may have a substituent and the like can be illustrated. As a halogen atom of the leaving group, a bromine atom or an iodine atom is preferable. As a lower alkyl group, a methyl group is preferable. As a halo-lower alkylsulfonyloxy group, a trifluoromethyl group is preferable. As an arylsulfonyloxy group which may have a substituent, a benzensulfonyloxy group or a toluenesulfonyloxy group is preferable.

As a 2-fluorobenzonitrile derivative having a leaving group at 4-position, a commercially available one or one prepared by a well-known method or optionally modified the same can be illustrated.

In the present process, first a Grignard reagent is prepared by mixing halophenol derivative (1) and a metallic magnesium in an inert solvent. As a solvent, for example, an ether solvent such as tetrahydrofuran, dimethoxyethane or the like, and a mixed solvent of an ether solvent and an aromatic hydrocarbon solvent such as toluene or the like can be illustrated. The temperature is preferably room temperature to 90°C. As an initiating reagent, for example, an iodine atom, dibromoethane or the like may be used.

After that, 3-fluorobiphenylcarbonitrile derivative (2) can be prepared by allowing the obtained Grignard reagent to drop the solution of the 2-fluorobenzonitrile derivative having a leaving group at 4-position including a heavymetallic catalyst usually for 1 to 20 hours, preferably for 2 to 10 hours (Kumada reaction). As a solvent, for example, an ether solvent such as tetrahydrofuran, dimethoxyethane or the like, an aromatic hydrocarbon solvent such as toluene or the like, and a mixed solvent thereof can be illustrated. As a catalyst, for example, a heavy metallic catalyst such as zerovalent or bivalent palladium, nickel and the like can be illustrated. It is usual to use 0.5 to 10 mol%, preferably 5 mol% of the catalyst against the 2-fluorobenzonitrile derivative having the leaving group at 4-position. To improve the yield, a ligand such as triphenylphosphine or the like, or a zinc reagent such as 5 to 10 mol% of zinc chloride, zinc bromide, zinc iodide or the like may be optionally added.

The obtained 3-fluorobiphenylcarbonitrile derivative (2) can be used in Process 2 after conventional handling. In addition, it can be also purified by the usual method such as column chromatography, recrystallization or the like.

### (Process 2)

This is a process for preparing 3-fluorobiphenylcarboxylic acid derivative (3) by subjecting 3-fluorobiphenylcarbonitrile derivative (2) to hydrolysis using an alkali metal hydroxide or the like.

As an alkali metal hydroxide, for example, potassium hydroxide, sodium hydroxide, lithium hydroxide monohydrate or the like can be illustrated. It is preferable to use 3 to 6 equivalents of the alkali metal hydroxide against 3-fluorobiphenylcarbonitrile derivative (2). Asa solvent, for example, a mixed solvent of water and an alcohol solvent, water and a hydrosoluble ether solvent and the like can be illustrated. The reaction temperature is preferably 80°C or higher. As an alcohol of an alcohol solvent, a secondary alcohol such as 2-propanol, propylene glycol monomethylether or the like is preferable, and propylene glycol monomethylether is particularly preferable. As an ether of a hydrosoluble ether solvent, dimethoxyethane or methyltetrahydrofuran is preferable. In addition, this process can be conducted in two-phase system using water and a water-immiscible solvent. In this case, it is preferable to add 0.1 to 1 equivalents of a phase transfer catalyst such as tetrabutylammonium chloride or the like.

The prepared 3-fluorobiphenylcarboxylic acid derivative (3) can be used in Process 3 after conventional handling. In addition, it can be also purified by the usual method such as column chromatography, recrystallization or the like.

### (Process 3)

This is a process for preparing 3-substituted biphenylcarboxylic acid derivative (4) by allowing 3-fluorobiphenylcarboxylic acid derivative (3) to react with a lower alkyl metal reagent, a fluoro-lower alkyl metal reagent, a cycloalkyl metal reagent or an aryl metal reagent which may have a substituent.

As a Grignard reagent such as a lower alkyl magnesiumhalide, a fluoro-lower alkyl magnesium halide, a cycloalkyl magnesium halide or an aryl magnesium halide which may have a substituent, any of one prepared by allowing the corresponding lower alkyl halide, fluoro-lower alkyl halide or cycloalkyl halide to react with metallic magnesium in the usual way (direct method), one prepared by allowing the corresponding lower alkyl halide, fluoro-lower alkyl halide or cycloalkyl halide to react with a Grignard reagent via magnesium-halogen exchange, and commercially available one can be used. As a halogen atom, a chloride atom, a bromine atom or an iodide atom is preferable. It is preferable to use more than 3 equivalents of the Grignard reagent against 3-fluorobiphenylcarboxylic acid derivative (3).

This reaction can be also conducted after changing 3-fluorobiphenylcarboxylic acid derivative (3) into an alkali metal salt thereof. As an alkali metal salt, a lithium salt is preferable. In this case, it is preferable to use more than 2 equivalents of the Grignard reagent against the alkali metal salt of 3-fluorobiphenylcarboxylic acid derivative (3).

As a lower alkyl lithium, a fluoro-lower alkyl lithium or a cycloalkyl lithium, one prepared by allowing the corresponding lower alkyl halide, fluoro-lower alkyl halide or cycloalkyl halide, or the corresponding lower alkyl thioether, fluoro-lower alkyl thioether or cycloalkyl thioether to react with a metallic lithium in the usual way can be used. As an aryl lithium reagent which may have a substituent, one prepared by subjecting an aryl bromide to exchange-reaction with an alkyl lithium can be used. Commercially available ones can be also used. As a halogen atom, a chloride atom or a bromide atom is preferable. It is preferable to use more than 3 equivalents of the lithium reagent against 3-fluorobiphenylcarboxylic acid derivative (3).

An organocopper reagent is usually called the Gilman reagent. The Gilman reagent can be prepared by mixing 2 equivalents of an organolithium reagent and 1 equivalent of a copper salt. As a copper salt, a copper halide (I), copper cyanide or the like can be used. It is preferable to use more than 3 equivalents of the copper salt against 3-fluorobiphenylcarboxylic acid derivative (3).

Among these organometallic reagents, a Grignard reagent, particularly, a sterically-bulky Grignard reagent such as isopropyl magnesium chloride, isobutyl magnesium chloride or the like is preferable. The reaction temperature is preferably 0 to 50°C.

The prepared 3-substituted biphenylcarboxylic acid derivative (4) can be directly used in Process 4 after conventional handling. In addition, it can be also purified by the usual method such as column chromatography, recrystallization or the like.

### (Process 4)

This is a process for preparing 3-substituted biphenylcarboxylate (5) by subjecting 3-substituted biphenylcarboxylic acid derivative (4) to esterification.

The esterification may be conducted by either method of allowing 3-substituted biphenylcarboxylic acid derivative (4) to react with a lower alkyl halide or a lower alkyl sulfate ester, or allowing 3-substituted biphenylcarboxylic acid derivative (4) to react with a lower alcohol.

In the method of allowing 3-substituted biphenylcarboxylic acid derivative (4) to react with a lower alkyl halide or a lower alkyl sulfate ester, it is preferable to mix 1.1 to 2 equivalents of a base of an alkali metal carbonate such as potassium carbonate or the like, or an alkali metal hydroxide such as sodium hydroxide or the like together. As a solvent, for example, an amide solvent such as N,N-dimethylformamide, N-methylpyrrolidone or the like; an ether solvent such as tetrahydrofuran or the like; an acetic ester solvent; a nitrile solvent such as acetonitrile or the like; an aromatic hydrocarbon solvent such as toluene or the like; or a mixed solvent of two or more of these solvents can be illustrated.

In the method of allowing 3-substituted biphenylcarboxylic acid derivative (4) to react with a lower alcohol, esterification is usually conducted with 0.1 to 2 equivalents of an inorganic acid such as hydrochloric acid, sulfuric acid or the like; a carboxylic acid such as oxalic acid or the like; or a sulfonic acid such as p-toluenesulfonic acid or the like in a lower alcohol as a solvent. Esterification can be also conducted by subjecting 3-substituted biphenylcarboxylic acid derivative (4) to condensation with a lower alcohol using a dehydration agent such as dicyclohexylcarbodiimide or the like. Furthermore, after 3-substituted biphenylcarboxylic acid derivative (4) is converted into an acid chloride by treatment with thionyl chloride or the like, esterification can be also conducted by reaction with a lower alcohol.

The prepared 3-substituted biphenylcarboxylate (5) can be directly used in Process 5 after conventional handling. In addition, it can be also purified by the usual method such as column chromatography, recrystallization or the like.

### (Process 5)

This is a process for preparing 4'-hydroxybiphenylcarboxylate (6) by the removal of a hydroxy-protective group of 3-substituted biphenylcarboxylate (5).

A condition of the removal of a hydroxy-protective group may be suitably decided in accordance with a kind of the protective group or a kind of the ester (see Protective Groups in Organic Synthesis (published by JOHON WILEY & SONS INC.) written by T W.Green & P G. M. Wuts). In case that a protective group is a benzyl group which may have a substituent, a hydrocracking reaction using palladium-carbon or the like is preferable, in case of a silyl group such as a triethylsilyl group or the like, a cleavage reaction using a fluoride reagent such as hydrofluoric acid or the like is preferable, and in case of a group forming a straight-chained or cyclic acetal such as a tetrahydropyranyl group or the like, a hydrolysis reaction using an acid such as hydrochloric acid or the like is preferable.

The prepared 4'-hydroxybiphenylcarboxylate (6) can be directly used in Process 6 after conventional handling. In addition, it can be also purified by the usual method such as column chromatography, recrystallization or the like.

### (Process 6)

This is a process for preparing 4'-epoxypropoxybiphenylcarboxylate (7) by allowing 4'-hydroxybiphenylcarboxylate (6) to react with an epihalohydrin.

As an epihalohydrin, for example, epibromohydrin and epichlorohydrin can be illustrated, and epibromohydrin is preferable. It is usual to use 1.2 to 3 equivalents of the epihalohydrin against 4'-hydroxybiphenylcarboxylate (6).

4'-Hydroxybiphenylcarboxylate (6) is allowed to react with epihalohydrin by mixing a base of an alkali metal carbonate such as potassium carbonate or the like, or an alkali metal hydroxide such as sodium hydroxide or the like at room temperature to 90°C for 2 to 24 hours. It is preferable to use 1.1 to 2 equivalents of the base against 4'-hydroxybiphenylcarboxylate (6). As a solvent, for example, an amide solvent such as N,N-dimethylformamide, N-methylpyrrolidone or the like; an ether solvent such as tetrahydrofuran or the like; an acetic ester solvent; a nitrile solvent such as acetonitrile or the like; an aromatic hydrocarbon solvent such as toluene or the like; or a mixed solvent of two or more of these solvents can be illustrated.

Since the prepared 4'-epoxyprpoxybiphenylcarboxylate (7) is well crystallized, it can be recrystallized by combining various solvents. As a rich solvent, an amide solvent such as N, N-dimethylformamide or the like; a sulfoxide solvent such as sulfolane, dimethylsulfoxide or the like; an acetic ester solvent; a nitrile solvent such as acetonitrile or the like; and an ether solvent such as tetrahydrofuran or the like can be illustrated. As a poor solvent, an alcohol solvent; and a hydrocarbon solvent such as toluene, *n*-heptane or the like; and water can be illustrated. In addition, it is possible that the recrystallization is conducted even though the alcohol solvent is used alone.

### (Process 7)

This is a process for preparing 4'-dihydroxypropoxybiphenylcarboxylate (8) by cleavage reaction of 4'-epoxypropoxybiphenylcarboxylate (7) to form a diol.

Cleavage reaction of 4'-epoxypropoxybiphenylcarboxylate (7) to form a diol can be conducted under either condition of an acidic condition or a strong basic condition in water and/or an organic solvent. As an organic solvent, a hydrosoluble solvent such as acetonitrile, acetone, methylethylketone, tetrahydrofuran, dimethoxyethane or the like is preferable.

As an acid, for example, sulfuric acid, phosphoric acid, perchloric acid or the like, or a solid superacid such as Nafion-H can be illustrated. In addition, as a base, for example, an alkali metal hydroxide such as sodium hydroxide or the like, tetrabutylammonium hydroxide and the like can be illustrated.

The prepared 4'-dihydroxypropoxybiphenylcarboxylate (8) can be directly used in Process 8 after conventional handling. In addition, it can be also purified by the usual method such as column chromatography, recrystallization or the like.

### (Process 8)

This is a process for preparing biphenyloxyacetoaldehyde derivative (9) by subjecting an adjacent diol moiety of 4'-dihydroxypropoxybiphenylcarboxylate (8) to oxidative cleavage.

As an oxidation agent, for example, a periodate, lead tetracetate, iodine-mercury(II) oxide, calcium hypochlorite-acetic acid, pyridinium chlorochromate, cerium ammonium nitrate or the like can be illustrated. In addition, oxidation can be conducted by electrolytic oxidation. Among these, a periodate, particularly an alkali metal periodate is preferable.

Oxidation using a periodate is preferably conducted usually at 1 to 50°C under a neutral condition or an acidic condition. As a solvent, for example, a hydrosoluble solvent such as acetonitrile, acetone, methylethylketone or the like; a hydrocarbon solvent such as toluene or the like; an ether solvent such as tert-butylmethylether or the like; an acetic ester solvent; a halogen-included hydrocarbon solvent such as chlorobenzene; and/or a mixed solvent with a carboxylic acid solvent such as acetic acid can be illustrated, it is preferable that the solvent contains water.

The prepared biphenyloxyacetoaldehyde derivative (9) can be directly used in reductive amination after conventional handling. In addition, it can be also purified by the usual method such as column chromatography or the like.

Aminoalcohol derivative (I) having a biphenyl group can be prepared by subjecting biphenyloxyacetoaldehyde derivative (9) obtained by the above method to reductive amination with aminoalcohol derivative (II), and subsequently by hydrolysis.

Reductive amination can be conducted by allowing biphenyloxyacetoaldehyde derivative (9) to react with aminoalcohol derivative (II) in an inert solvent at 0 to 60°C for 1 to 5 hours, and subsequently by allowing the mixture to react in the presence of a reducing agent at 0 to 60°C for 1 to 48 hours. As a reducing agent, for example, a boron reducing agent such as sodium borohydride, sodium triacetoxyborohydride, sodium cyanoborohydride or the like; and an aluminous reducing agent such as diisobutylaluminiumhydride or the like can be illustrated. A reducing agent may be used alone or in combination with an acid such as acetic acid or the like or a base such as trimethylamine or the like. In addition, reduction can be conducted by mixing with a catalyst such as 5 to 10% palladium-carbon or platinum oxide under a hydrogen atmosphere. In this case, it is preferable to use a catalyst which does not reduce a biphenylcarboxylate. The obtained biphenylcarboxylate can be converted into aminoalcohol derivative (I) by any method which is usually used in hydrolysis of an ester. The prepared aminoalcohol derivative (I) can be isolated by conducting the usual handling.

### Examples

The present invention is further illustrated inmore detail by way of the following Examples, however the invention is not limited thereto.

### Example 1

### 4'-Benzyloxy-3-fluoro-3',5'-dimethylbiphenyl-4-carbonitrile

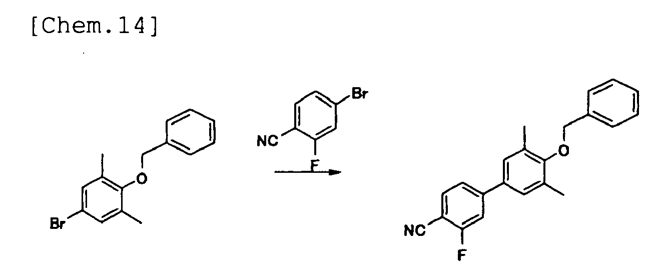

To magnesium (46.8 g) and tetrahydrofuran (800 mL), iodine (2.2g) and 1-benzyloxy-2,6-dimethyl-4-bromobenzene (50 g) were added at room temperature. The mixture was stirred at 50 °C and confirmed starting of the reaction. In addition, a solution of 1-benzyloxy-2,6-dimethyl-4-bromobenzene (460 g) in tetrahydrofuran (2.0 L) was added dropwise to the mixture at same temperature and stirred at 75 °C for an hour to prepare the Grignard reagent.

In another vessel, 2-fluoro-4-bromobenzonitrile (250 g), bis (triphenylphosphine) palladium (II) dichloride (4.39 g) and zinc chloride (8.52 g) were added to tetrahydrofuran (560 mL) at room temperature successively. After the mixture was stirred at room temperature for 15 minutes, the preliminary-prepared Grignard reagent was added dropwise to the mixture at internal temperature from 20 to 30°C for more than 2 hours and stirred at room temperature for an hour. The mixture was diluted with toluene and added 2 mol/L hydrochloric acid. The separated organic layer was washed with water, 1.5% ethylenediamine aqueous solution, 2 mol/L hydrochloric acid, brine and saturated sodium bicarbonate aqueous solution successively, and dried over anhydrous magnesium sulfate. The filtrate was concentrated under reduced pressure. The residue was dissolved in toluene and solvent was removed under reduced pressure once again. To the residue was added 2-propanol, and the precipitated crystals were collected by filtration to give 4'-benzyloxy-3-fluoro-3',5'-dimethylbiphenyl-4-carbonitrile (386 g, 93% yield).
¹H-NMR (CDCl₃) δ ppm:
2.36 (6H, s), 4.86 (2H, s), 7.25 (2H, s), 7.35-7.45 (5H, m), 7.49 (2H, d, J=7.0Hz), 7.64 (1H, dd, J=7.0, 8.0Hz).

### Example 2

### 4'-Benzyloxy-3-fluoro-3',5'-dimethylbiphenyl-4-carboxylic acid

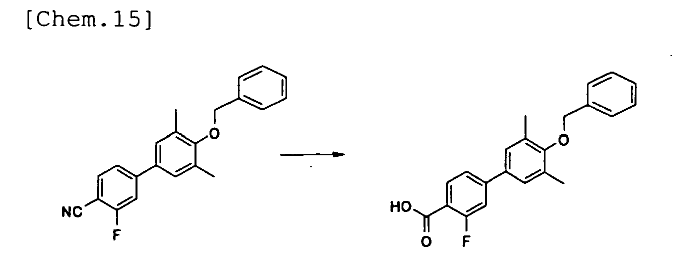

To a solution of lithium hydroxide monohydrate (241 g) in water (1.1 L), 4'-benzyloxy-3-fluoro-3',5'-dimethylbiphenyl-4-carbonitrile(380 g) and propylene glycol monomethylether, (0.62 L) were added and the mixture was stirred at reflux temperature for 12 hours. The mixture of tetrahydrofuran and water was added to the reaction mixture. In addition, 2 mol/L hydrochloric acid (3.15 L) was added to the mixture under ice-cooling and the precipitated crystals were collected by filtration to give 4'-benzyloxy-3-fluoro-3',5'-dimethylbiphenyl-4-carboxylic acid (361 g, 90% yield).
¹H-NMR (CDCl₃) δ ppm:
2.37 (6H, s), 4.87 (2H, s), 7.31 (2H, s), 7.35-7.46 (5H, m), 7.50 (2H, d, J=7.0Hz), 8.07 (1H, t, J=7.5Hz).

### Example 3

### 4'-Benzyloxy-3-isopropyl-3',5'-dimethylbiphenyl-4-carboxylic acid

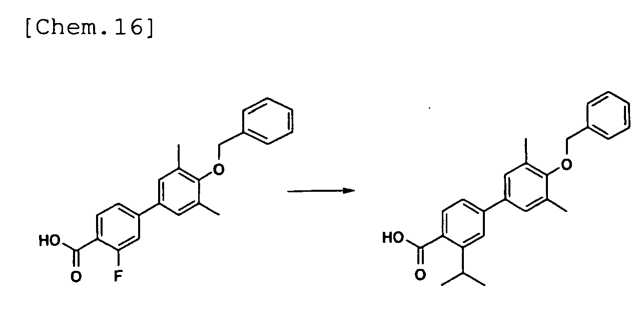

To a solution of 4'-benzyloxy-3-fluoro-3',5'-dimethybiphenyl-4-carboxylic acid (320 g) in tetrahydrofuran (1.4 L), 2 mol/L solution (1.5 L) of isopropylmagnesium chloride in tetrahydrofuran was added under ice-cooling, and the mixture was stirred at 40°C for 2 hours. The reaction mixture was poured dropwise into 2 mol/L hydrochloric acid in another vessel under ice-cooling, and the separated organic layer was washed with 5% brine. The organic layer was diluted with ethyl acetate, and washed with 20% brine. The organic layer was concentrated under reduced pressure to give 4' -benzyloxy-3-isopropyl-3',5' - dimethylbiphenyl-4-carboxylic acid (342 g, 100% yield) as a soild.
¹H-NMR (DMSO-d₆) δ ppm:
1.27 (6H, d, J=7.0Hz), 2.33 (6H, s), 3.79-3.85 (1H, m), 4.83 (2H, s), 7.36-7.43 (5H, m), 7.49-7.53 (2H, m), 7.65 (1H, d, J=1.7Hz), 7.73 (1H, d, J=8.2Hz), 12.9 (1H, br-s).

### Example 3

### Methyl 4'-benzyloxy-3-isopropyl-3',5'-dimethylbiphenyl-4-carboxylate

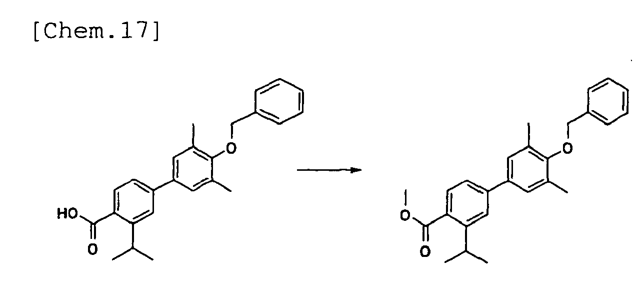

To a suspension of 4'-benzyloxy-3-isopropyl-3',5'-dimethylbiphenyl-4-carboxylic acid (342 g) and potassium carbonate (189 g) in 1-methyl-2-pyrrolidone (1.2 L), dimethyl sulfate (130 mL) was added dropwise under ice-cooling, and the mixture was stirred at the same temperature for an hour. In addition, after the mixture was stirred at 50°C for an hour, water was added dropwise to the reaction mixture under ice-brine bath, and the precipitated crystals were collected by filtration. The obtained crystals were dissolved in 2-propanol, and the precipitated crystals were collected by filtration to give methyl 4'-benzyloxy-3-isopropyl-3',5'-dimethylbiphenyl-4-carboxylate (298 g, 84% yield).
¹H-NMR (CDCl₃) δ ppm:
1.32 (6H, d, J=7.0Hz), 2.37 (6H, s), 3.80-3.86 (1H, m), 3.91 (3H, s), 4.86 (2H, s), 7.27 (2H, s), 7.35-7,45 (4H, m), 7.51 (2H, d, J=7.5Hz), 7.58 (1H, d, J=1.5Hz), 7.81 (1H, d, J=8.0Hz).

### Example 4

### Methyl 4'-hydroxy-3-isopropyl-3',5'-dimethylbiphenyl-4-carboxylate

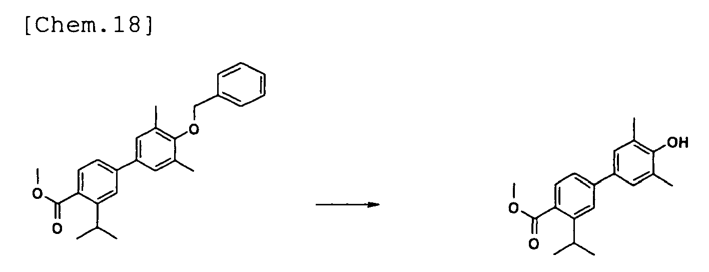

A suspension of methyl 4'-benzyloxy-3-isopropyl-3',5'-dimethylbiphenyl-4-carboxylate (200 g) and 10% palladium-carbon (56wt% wet) (28 g) in tetrahydrofuran (1400 g) was stirred at room temperature for 3 hours under a hydrogen atmosphere. After the catalyst was removed by filtration, the filtrate was concentrated under reduced pressure to give methyl 4'-hydroxy-3-isopropyl-3',5'-dimethylbiphenyl-4-carboxylate (162 g, quantitative yield).
¹H-NMR (CDCl₃) δ ppm:
1.31 (6H, d, J=6.9Hz), 2.33 (6H, s), 3.80-3.87 (1H, m), 3.90 (3H, s), 4.82 (1H, br-s), 7.23 (2H, s), 7.37 (1H, dd, J=1.8, 8.0Hz), 7.55 (1H, d, J=1.8Hz), 7.80 (1H, d, J=8.0Hz).

### Example 5

### Methyl 3-isopropyl-3',5'-dimethyl-4'-oxiranylmethyloxybiphenyl-4-carboxylate

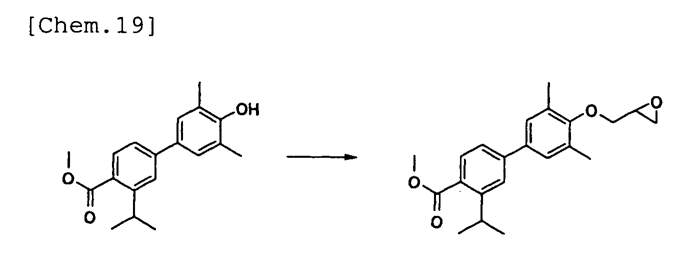

A suspension of methyl 4'-hydroxy-3-isopropyl-3',5'-dimethylbiphenyl-4-carboxylate (154 g) and potassium carbonate (142 g) in N,N-dimethylformamide (400 g) was stirred at room temperature for an hour. Epibromohydrin (66 mL) was added to the reaction mixture at room temperature. After stirring for 15 minutes, the mixture was stirred at internal temperature from 64 to 70°C for 15 hours. The mixture was diluted with ethyl acetate and washed with water. The organic layer was concentrated under reduced pressure and the residue was dissolved in diisopropyl ether. The solution was concentrated under reduced pressure once again. The mixture solvent of diisopropyl ether and 2-propanol was added to the residue and the precipitated crystals were collected by filtration to give methyl 3-isopropyl-3',5'-dimethyl-4'-oxiranylmethyloxybiphenyl-4-carboxylate (153 g, 84% yield).
¹H-NMR (CDCl₃) δ ppm:
1.31 (6H, d, J=6.8Hz), 2.38 (6H, s), 2.74 (1H, dd, J=2.7, 4.9Hz), 2.90-2.93 (1H, m), 3.38-3.42 (1H, m), 3.75-3.90 (2H, m), 3.91 (3H, s), 4.10 (1H, dd, J=3.2, 11Hz), 7.25 (2H, s), 7.38 (1H, dd, J=1.9, 8.1Hz), 7.55 (1H, d, J=1.9Hz), 7.80 (1H, d, J=8.1Hz).

### Example 6

### Methyl 4'-(2,3-dihydroxypropyl)oxy-3-isopropyl-3',5'-dimethylbiphenyl-4-carboxylate

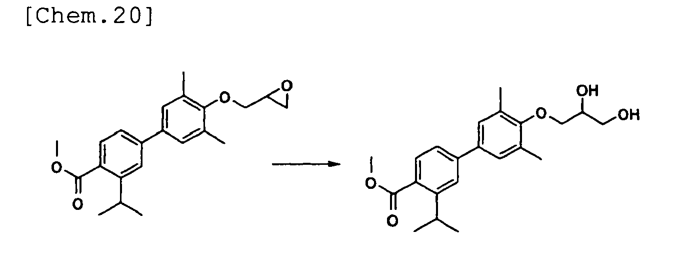

To a suspension of methyl 3-isopropyl-3',5'-dimethyl-4'-oxiranylmethyloxybiphenyl-4-carboxylate (120 g) in acetonitrile (120 g) was added 6.4% aqueous solution of perchloric acid (275 g), and the mixture was stirred at 40°C for 6 hours. After 20% aqueous solution of potassium carbonate was added to the mixture at room temperature, the separated organic layer was washed with 15% brine. The organic layer was concentrated under reduced pressure and the residue was dissolved in acetonitrile, and then the solvent was removed under reduced pressure to give methyl 4'-(2,3-dihydroxypropyl)oxy-3-isopropyl-3',5'-dimethylbiphenyl-4-carboxylate (128 g, quantitative yield).
¹H-NMR (CDCl₃) δ ppm:
1.31 (6H, d, J=7.1Hz), 2.36 (6H, s), 3.80-3.92 (8H, m), 4.10-4.20 (1H, m), 7.25(2H, s), 7.37 (1H, dd, J=1.5, 8.2Hz), 7.55 (1H, d, J=1.5Hz), 7.79 (1H, d, J=8.2Hz).

### Example 7

### Methyl 4'-formylmethyloxy-3-isopropyl-3',5'-dimethylbiphenyl-4-carboxylate

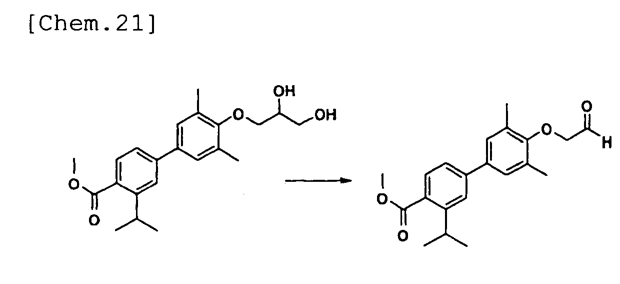

To a solution of methyl 4'-(2,3-dihydroxypropyl)oxy-3-isopropyl-3',5'-dimethylbiphenyl-4-carboxylate (126 g) in acetonitrile (360g) was added an solution of sodium metaperiodate (87 g) in water (1.0 L) and tert-butyl methyl ether (200 mL) successively at room temperature. After the mixture was stirred at room temperature for 15 minutes, it was stirred at 40°C for 16 hours. Water was added to the mixture at room temperature, and the separated organic layer was washed with water and 20% brine. The organic layer was concentrated under reduced pressure. The residue was dissolved in tetrahydrofuran and the solvent was removed under reduced pressure once again to give methyl 4'-formylmethyloxy-3-isopropyl-3',5'-dimethylbiphenyl-4-carboxylate (122g, 106% yield, 92% purity).
¹H-NMR (CDCl₃) δ ppm:
1.31 (6H, d, J=6.8Hz), 2.37 (6H, s), 3.80-3.90 (1H, m), 3.91 (3H, s), 4.45 (2H, d, J=0.8Hz), 7.26(2H, s), 7.38 (1H, dd, J=1.6, 8.0Hz), 7.55 (1H, d, J=1.6Hz), 7.80 (1H, d, J=8.0Hz), 9.98 (1H, t, J=0.8Hz).

### Example 8

### Methyl (-)-4'-[2-[[(1S,2R)-2-hydroxy-2-(4-hydroxyphenyl)-1-methylethyl]amino]ethoxy]-3-isopropyl-3',5'-dimethylbiphenylcarboxylate hydrochloride

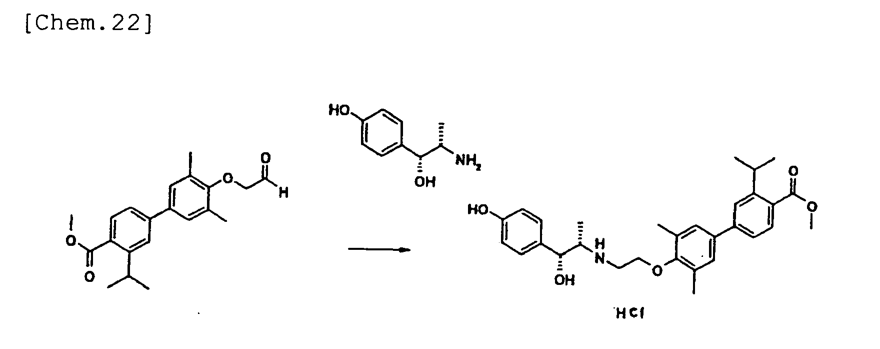

To a solution of methyl 4'-formylmethyloxy-3-isopropyl-3',5'-dimethylbiphenyl-4-carboxylate(40g)in tetrahydrofuran (200 g) was added (1R,2S)-2-amino-1-(4-hydroxyphenyl)-propane-1-ol (20g), and the mixture was stirred at 40°C for an hour. The mixture was concentrated under reduced pressure, and tetrahydrofuran (200 g) was added to the mixture to give a solution of imine.

To a suspension of sodium triacetoxyborohydrate (76.1 g) in tetrahydrofuran (400 g) in another vessel, the solution of the prepared imine at 40 to 45°C was added dropwise, and the mixture was stirred at same temperature for 2 hours. A solution of triethanolamine (21.4 g) in water (40 mL) was added to the mixture, and the mixture was stirred at 60 °C for 30 minutes. After 20% aqueous solution of potassium carbonate was added to the mixture at room temperature, and the mixture was stirred for 30 minutes. The separated organic layer was washed with 10% brine, and the organic layer was concentrated under reduced pressure. The residue was diluted with ethyl acetate, and washed with 20% brine. The organic layer dried over anhydrous magnesium sulfate and the filtrate was concentrated under reduced pressure. The residue was dissolved in ethanol (20 mL) and ethyl acetate (400 mL). Four mol/L hydrogen chloride solution (54 mL) in ethyl acetate was added to the mixture at room temperature, and the precipitated crystals were collected by filtration to give methyl (-)-4'-[2-[[(1S,2R)-2-hydroxy-2-(4-hydroxyphenyl)-1-methylethyl]amino]ethoxy-3-isopropyl-3',5'-dimethylbiphenylcarboxylate hydrochloride (53g, 83% yield). It is possible to convert this compound into (-)-4'-[2-[[(1S,2R)-2-hydroxy-2-(4-hydroxyphenyl)-1-methylethyl]amino]ethoxy-3-isopropyl-3',5'-dimethylbiphenylcarboxylic acid by the usual hydrolysis easily.
¹H-NMR (DMSO-d₆) δ ppm:
1.03 (3H, d, J=6.5Hz), 1.27 (6H, d, J=6.6Hz), 2.36 (6H, s), 3.40-3.55 (3H, br), 3.64-3.78 (1H, m), 3.84 (3H, s), 4.05-4.20 (2H, br), 5.15 (1H, br-s), 5.98 (1H, br-s), 6.78 (2H, d, J=8.5Hz), 7.19 (2H, d, J=8.5Hz), 7.43 (1H, s), 7.52-7.54 (1H, m), 7.66 (1H, d, J=1.6Hz), 7.72 (1H, d, J=8.0Hz), 8.80-9.05 (2H, br), 9.41 (1H, s).

## Claims

1. A method for preparing biphenyloxyacetoaldehyde derivative (9) represented by the general formula: 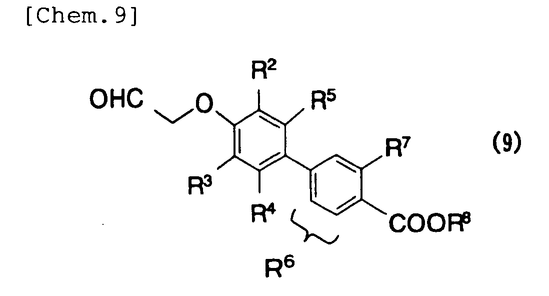 wherein R² to R⁵ represent independently a hydrogen atom, a fluorine atom, a chlorine atom, a lower alkyl group, a fluoro-lower alkyl group or a lower alkoxy group, R⁶ represents a fluorine atom, a chlorine atom, a lower alkyl group, a fluoro-lower alkyl group, a cycloalkyl group, a lower alkoxy group or an aryloxy group which may have a substituent, R⁷ represents a lower alkyl group, a fluoro-lower alkyl group, a cycloalkyl group or an aryl group which may have a substituent, R⁸ represents a lower alkyl group or an arylmethy group which may have a substituent, and "}R⁶" indicates that R⁶ exists at m-position and/or p-position of R ⁷ (when two R⁶ exist, these may be different), which comprises:
Process 1 for preparing 3-fluorobiphenylcarbonitrile derivative (2) represented by the general formula: 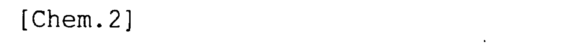 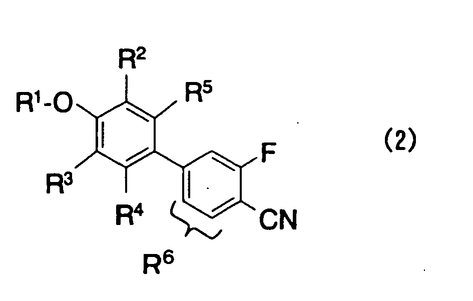 wherein R¹ represents a hydroxy-protective group, R² to R⁶ have the same meanings as defined above, and "}R⁶" indicates that R⁶ exists at m-position and/or p-position of the fluorine atom and when two R⁶ exist, these may be different, which comprises by allowing halophenol derivative (1) represented by the following general formula: 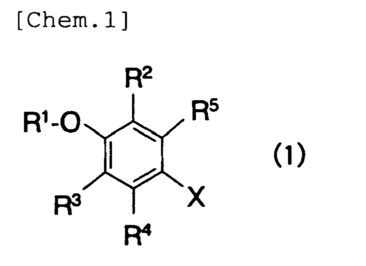 wherein R¹ to R⁵ have the same meanings as defined above, and X represents a chlorine atom, a bromine atom or an iodine atom, to react with a 2-fluorobenzonitrile derivative which is substituted by a leaving group at 4-position;
Process 2 for preparing 3-fluorobiphenylcarboxylic acid derivative (3) represented by the general formula: 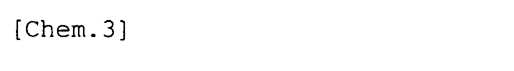 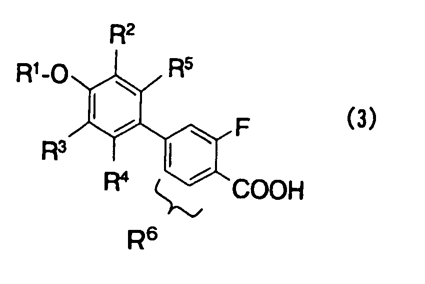 wherein R¹ to R⁶ have the same meanings as defined above, by hydrolysis of 3-fluorobiphenylcarbonitrile derivative (2);
Process 3 for preparing 3-substituted biphenylcarboxylic acid derivative (4) represented by the general formula: 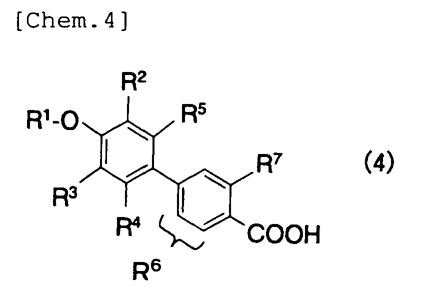 wherein R¹ to R⁷ have the same meanings as defined above, by allowing 3-fluorobiphenylcarboxylic acid derivative (3) to react with a lower alkyl metal reagent, a fluoro-lower alkyl metal reagent, a cycloalkyl metal reagent or an aryl metal reagent which may have a substituent;
Process 4 for preparing 3-substituted biphenylcarboxylate (5) represented by the general formula: 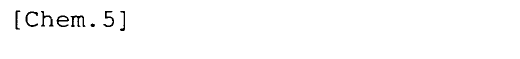 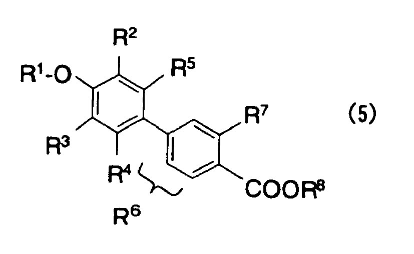 wherein R¹ to R⁸ have the same meanings as defined above, by esterification of 3-substituted biphenylcarboxylic acid derivative (4);
Process 5 for preparing 4'-hydroxybiphenylcarboxylate (6) represented by the general formula: 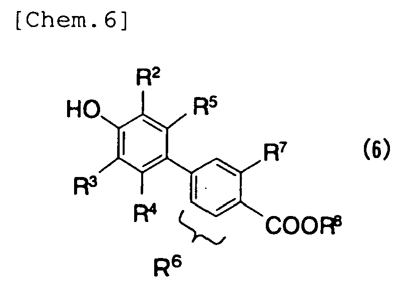 wherein R² to R⁸ have the same meanings as defined above, by removal of a hydroxy-protective group of 3-substituted biphenylcarboxylate (5);
Process 6 for preparing 4'-epoxypropoxybiphenylcarboxylate (7) represented by the general formula: 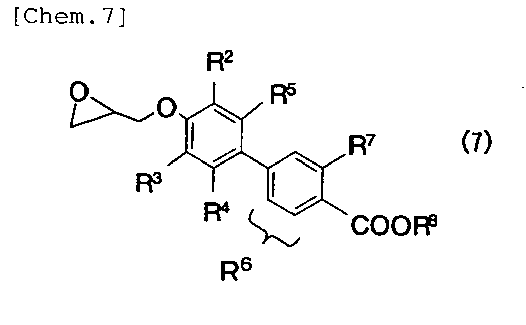 wherein R² to R⁸ have the same meanings as defined above, by allowing 4'-hydroxybiphenylcarboxylate (6) to react with an epihalohydrin;
Process 7 for preparing 4'-dihydroxypropoxybiphenylcarboxylate (8) represented by the general formula: 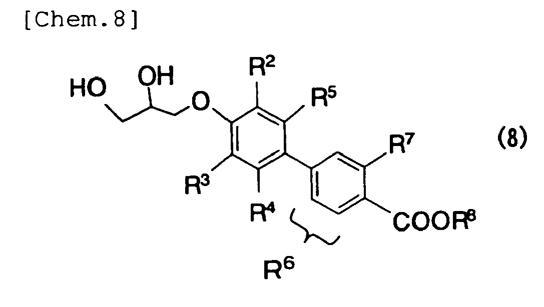 wherein R² to R⁸ have the same meanings as defined above, by cleavage reaction of 4'-epoxypropoxybiphenylcarboxylate (7) to form a diol; and
Process 8 for preparing biphenyloxyacetoaldehyde derivative (9) by oxidation of 4'-dihydroxypropoxybiphenylcarboxylate (8).

2. A method for preparing aminoalcohol derivative (I): 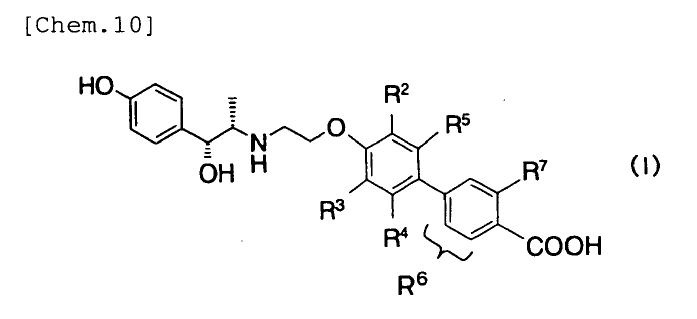 wherein R² to R⁵ represent independently a hydrogen atom, a fluorine atom, a chlorine atom, a lower alkyl group, a fluoro-lower alkyl group or a lower alkoxy group, R⁶ represents a fluorine atom, a chlorine atom, a lower alkyl group, a fluoro-lower alkyl group, a cycloalkyl group, a lower alkoxy group or an aryloxy group which may have a substituent, and R ⁷ represents a lower alkyl group, a fluoro-lower alkyl group, a cycloalkyl group or an aryl group which may have a substituent, and"}R⁶" indicates that R⁶ exists at m-position and/ or p-position of R⁷ (when two R⁶ exist, these may be different), which comprises subjecting biphenyloxyacetoaldehyde derivative (9) represented by the following general formula: 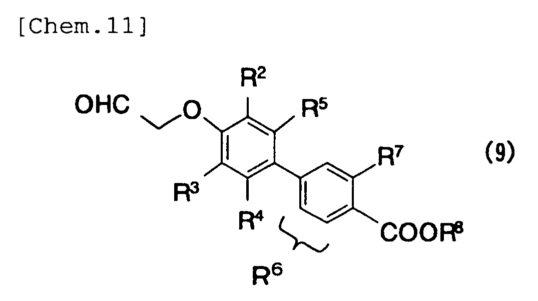 wherein R² to R⁷ have the same meanings as defined above, and R⁸ represents a lower alkyl group or an arylmethyl group which may have a substituent, to reductive amination with aminoalcohol derivative (II) represented by the following general formula: 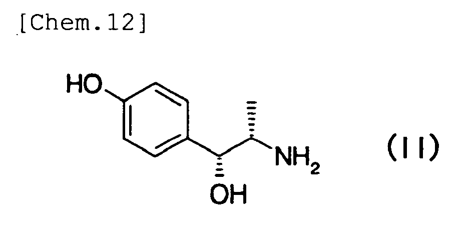 , and subsequently by hydrolysis.

3. A3-fluorobiphenylcarbonitrile derivative represented by the general formula (2): 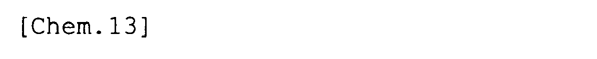 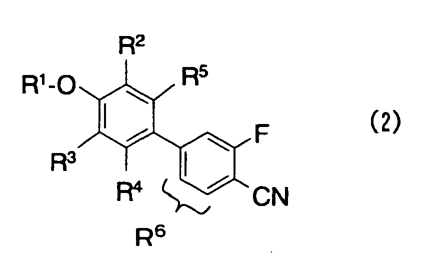 wherein R¹ represents a hydroxy-protective group, R² to R⁵ represent independently a hydrogen atom, a fluorine atom, a chlorine atom, a lower alkyl group, a fluoro-lower alkyl group or a lower alkoxy group, R⁶ represents a fluorine atom, a chlorine atom, a lower alkyl group, a fluoro-lower alkyl group, a cycloalkyl group, a lower alkoxy group or an aryloxy group which may have a substituent, and "}R⁶" indicates that R⁶ exists at m-position and/or p-position of the fluorine atom (when two R⁶ exist, these may be different).

4. A3-fluorobiphenylcarboxylic acid derivative represented by the general formula (3): 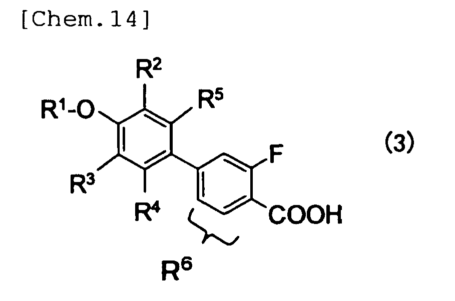 wherein R¹ represents a hydroxy-protective group, R² to R⁵ represent independently a hydrogen atom, a fluorine atom, a chlorine atom, a lower alkyl group, a fluoro-lower alkyl group or a lower alkoxy group; R⁶ represents a fluorine atom, a chlorine atom, a lower alkyl group, a fluoro-lower alkyl group, a cycloalkyl group, a lower alkoxy group or an aryloxy group which may have a substituent, and "}R⁶" indicates that R⁶ exists at m-position and/or p-position of the fluorine atom (when two R⁶ exist, these may be different).

5. A 3-substituted biphenylcarboxylic acid derivative represented by the general formula (4): 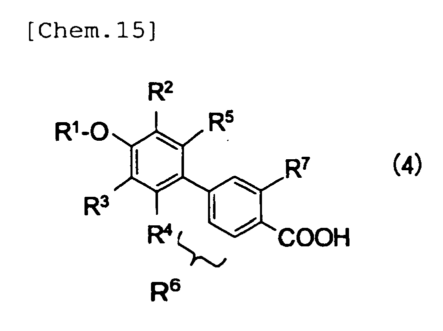 wherein R¹ represents a hydroxy-protective group, R² to R⁵ represent independently a hydrogen atom, a fluorine atom, a chlorine atom, a lower alkyl group, a fluoro-lower alkyl group or a lower alkoxy group, R⁶ represents a fluorine atom, a chlorine atom, a lower alkyl group, a fluoro-lower alkyl group, a cycloalkyl group, a lower alkoxy group or an aryloxy group which may have a substituent, R⁷ represents a lower alkyl group, a fluoro-lower alkyl group, a cycloalkyl group or an aryl group which may have a substituent, and "}R⁶" indicates that R⁶ exists at m-position and/or p-position of R⁷ (when two R⁶ exist, these may be different).

6. A 3-substituted alkylbiphenylcarboxylate represented by the general formula: 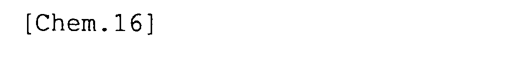 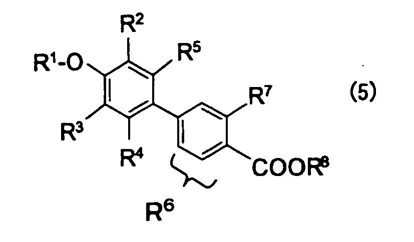 wherein R represents a hydroxy-protective group, R to R⁵ represent independently a hydrogen atom, a fluorine atom, a chlorine atom, a lower alkyl group, a fluoro-lower alkyl group or a lower alkoxy group, R⁶ represents a fluorine atom, a chlorine atom, a lower alkyl group, a fluoro-lower alkyl group, a cycloalkyl group, a lower alkoxy group or an aryloxy group which may have a substituent, R⁷ represents a lower alkyl group, a fluoro-lower alkyl group, a cycloalkyl group or an aryl group which may have a substituent, R⁸ represents a lower alkyl group or an arylmethyl group which may have a substituent, and "}R⁶" indicates that R⁶ exists at m-position and/or p-position of R⁷ (when two R⁶ exist, these may be different).

7. A 4'-hydroxybiphenylcarboxylate represented by the general formula: 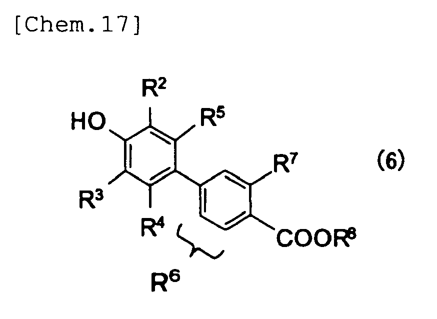 wherein R² to R⁵ represent independently a hydrogen atom, a fluorine atom, a chlorine atom, a lower alkyl group, a fluoro-lower alkyl group or a lower alkoxy group, R⁶ represents a fluorine atom, a chlorine atom, a lower alkyl group, a fluoro-lower alkyl group, a cycloalkyl group, a lower alkoxy group or an aryloxy group which may have a substituent, R⁷ represents a lower alkyl group, a fluoro-lower alkyl group, a cycloalkyl group or an aryl group which may have a substituent, R⁸ represents a lower alkyl group or an arylmethyl group which may have a substituent, and "}R⁶" indicates that R⁶ exists at m-position and/or p-position of R⁷ (when two R⁶ exist, these may be different).

8. A 4'-epoxypropoxybiphenylcarboxylate represented by the general formula: 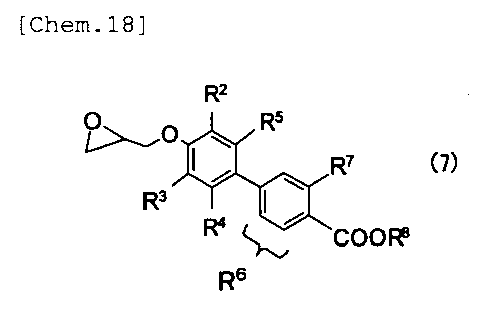 wherein R² to R⁵ represent independently a hydrogen atom, a fluorine atom, a chlorine atom, a lower alkyl group, a fluoro-lower alkyl group or a lower alkoxy group, R⁶ represents a fluorine atom, a chlorine atom, a lower alkyl group, a fluoro-lower alkyl group, a cycloalkyl group, a lower alkoxy group or an aryloxy group which may have a substituent, R⁷ represents a lower alkyl group, a fluoro-lower alkyl group, a cycloalkyl group or an aryl group which may have a substituent, R⁸ represents a lower alkyl group or an arylmethyl group which may have a substituent, and "}R⁶" indicates that R⁶ exists at m-position and/or p-position of R⁷ (when two R⁶ exist, these may be different).

9. A4'-dihydroxypropoxybiphenylcarboxylate representedby the general formula: 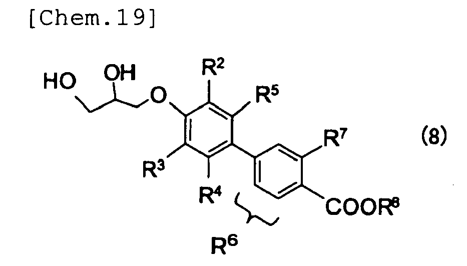 wherein R² to R⁵ represent independently a hydrogen atom, a fluorine atom, a chlorine atom, a lower alkyl group, a fluoro-lower alkyl group or a lower alkoxy group, R⁶ represents a fluorine atom, a chlorine atom, a lower alkyl group, a fluoro-lower alkyl group, a cycloalkyl group, a lower alkoxy group or an aryloxy group which may have a substituent, R⁷ represents a lower alkyl group, a fluoro-lower alkyl group, a cycloalkyl group or an aryl group which may have a substituent, R⁸ represents a lower alkyl group or an arylmethyl group which may have a substituent, and "}R⁶" indicates that R⁶ exists at m-position and/or p-position of R ⁷ (when two R⁶ exist, these may be different).

10. A biphenyloxyacetoaldehyde derivative represented by the general formula: 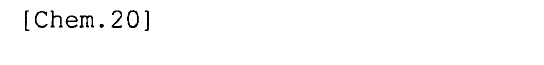 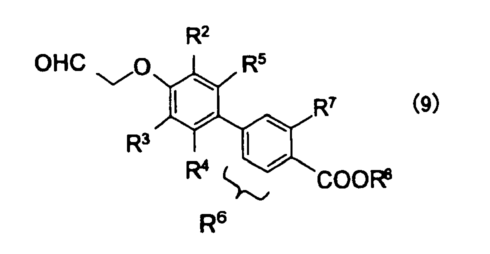 wherein R² to R⁵ represent independently a hydrogen atom, a fluorine atom, a chlorine atom, a lower alkyl group, a fluoro-lower alkyl group or a lower alkoxy group, R⁶ represents a fluorine atom, a chlorine atom, a lower alkyl group, a fluoro-lower alkyl group, a cycloalkyl group, a lower alkoxy group or an aryloxy group which may have a substituent, R⁷ represents a lower alkyl group, a fluoro-lower alkyl group, a cycloalkyl group or an aryl group which may have a substituent, R⁸ represents a lower alkyl group or an arylmethyl group which may have a substituent, and "}R⁶" indicates that R⁶ exists at m-position and/or p-position of R ⁷ (when two R⁶ exist, these may be different).
